(19) Europäisches Patentamt
European Patent Office
Office européen des brevets

(11) **EP 2 638 930 B1**

(12) **FASCICULE DE BREVET EUROPEEN**

(45) Date de publication et mention de la délivrance du brevet:
**20.08.2014 Bulletin 2014/34**

(51) Int Cl.:
***A61N 1/378*** *(2006.01)*     ***A61N 1/365*** *(2006.01)*

(21) Numéro de dépôt: **12183716.5**

(22) Date de dépôt: **10.09.2012**

(54) **Capsule intracorporelle autonome à double récupération d'énergie**

Autonome körperinterne Kapsel mit doppelter Energierückgewinnung

Autonomous intracorporeal capsule with dual energy recovery

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.03.2012 FR 1252216**

(43) Date de publication de la demande:
**18.09.2013 Bulletin 2013/38**

(73) Titulaire: **Sorin CRM SAS**
**92140 Clamart (FR)**

(72) Inventeurs:
• **Deterre, Martin**
**75013 Paris (FR)**
• **Makdissi, Alaa**
**75011 Paris (FR)**

(74) Mandataire: **Dupuis-Latour, Dominique et al**
**Bardehle Pagenberg**
**10, boulevard Haussmann**
**75009 Paris (FR)**

(56) Documents cités:
| DE-A1-102004 043 002 | US-A1- 2005 256 549 |
| US-A1- 2007 276 444 | US-A1- 2007 293 904 |
| US-A1- 2009 281 600 | US-A1- 2010 217 354 |

**Description**

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resyn-chronisation, de défibrillation et/ou de cardioversion en cas de trouble du rythme détecté par l'appareil. Elle inclut aussi les appareils neurologiques, les implants cochléaires, etc. ainsi que les dispositifs de mesure de pH ou encore d'impédance intracorporelle telle que l'impédance transpulmonaire ou l'impédance intracardiaque.

Les récentes avancées en matière de microfabrication et de biotechnologies permettent dorénavant le développement d'une grande variété de tels dispositifs implantables miniaturisés, offrant aux praticiens des procédures d'implantation moins invasives, plus de confort pour les patients, des performances accrues et qui souvent ouvrent l'accès à de nouveaux types de diagnostics et de traitements.

Les implants actifs traditionnels de type stimulateur cardiaque sont volumineux, délocalisés du coeur et doivent être reliés au site de stimulation par une sonde. A l'opposé, les possibilités de miniaturisation conduisent au développement de dispositifs si petits qu'ils peuvent être entièrement implantés sur site, par exemple directement dans une cavité cardiaque, tout en fonctionnant en parfaite autonomie.

L'invention concerne plus particulièrement ceux de ces dispositifs qui mettent en oeuvre des capsules autonomes implantées, dépourvues de toute liaison physique à un dispositif principal également implanté tel qu'un boîtier de générateur d'impulsions de stimulation.

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique une électrode ou un capteur à un générateur connecté à l'extrémité opposée, proximale, de la sonde.

De tels implants sans sonde ou capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces implants *leadless* ne se limitent pas aux stimulateurs entièrement autonomes. Ils peuvent être également des satellites sans fil de systèmes de stimulation plus complexes, par exemple comprenant un plus gros boitier implanté sous la peau, ou même de simples capteurs autonomes, relayant leurs données vers un système distant.

Les capsules *leadless* peuvent être par exemple des capsules épicardiques, fixées à la paroi extérieure du coeur, ou bien des capsules endocavitaires, fixées à la paroi intérieure d'une cavité ventriculaire ou auriculaire au moyen d'une vis d'ancrage saillante, prolongeant axialement le corps de la capsule et destinée à pénétrer dans le tissu cardiaque par vissage au site d'implantation.

L'invention est plus spécialement dédiée aux capsules endocavitaires.

Une capsule *leadless* comprend des circuits de détection/stimulation pour recueillir des potentiels de dépolarisation du myocarde et/ou pour appliquer des impulsions de stimulation au site où est implantée la capsule. Celle-ci porte alors une électrode appropriée, qui peut être notamment constituée par une partie active de la vis d'ancrage. Elle peut également incorporer, en variante ou en complément, un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient comme indicateur de l'activité, etc.

Bien entendu, les capsules *leadless* incorporent également des moyens émetteurs/récepteurs de communication sans fil pour l'échange de données à distance.

Quelle que soit la technique mise en oeuvre, le traitement des signaux au sein de la capsule et leur transmission à distance nécessitent une énergie non négligeable par rapport aux ressources énergétiques que peut stocker cette capsule. Or, compte tenu de son caractère autonome, la capsule ne peut faire appel qu'à ses ressources propres telles qu'un circuit de récupération d'énergie associé à une petite batterie tampon intégrée.

La source d'énergie est un donc un élément majeur des capsules intra-corporelles *leadless* dans la perspective d'une miniaturisation allant jusqu'à l'autonomie complètes des capsules, au sens où l'énergie ne leur est transmise par un aucun système auxiliaire.

Or, si l'énergie consommée par la capsule était fournie par une pile, la densité d'énergie des piles actuelles ne serait pas suffisante pour garantir une durée de vie de plusieurs années à une capsule miniaturisée.

Un des axes de recherche les plus prometteurs pour résoudre ce problème est de développer un module de récupération d'énergie, dont le volume est considérablement réduit par rapport à celui d'une pile classique, et dont la source d'énergie provient de l'environnement ambiant, typiquement sous forme mécanique (vibrations, déformations, mouvements, pression ...) ou autre (thermique, solaire, électromagnétique, chimique ...). La durée de vie d'une telle source d'énergie n'est donc plus limitée par sa capacité, mais uniquement par sa tenue mécanique et son vieillissement naturel.

Cependant, fournir assez de puissance de manière régulière pour alimenter la capsule tout en réduisant la taille du récupérateur d'énergie constitue une limitation technique majeure, et dépend entièrement de l'importance et de la nature de l'énergie que l'on souhaite récupérer dans l'environnement.

A l'exemple des US 6 984 902 et US 7 843 090, la majorité des systèmes de récupération d'énergie actuels sont basés sur un dispositif inertiel, c'est-à-dire qui utilise l'accélération du milieu ambiant pour agir sur une masse, dite "masse sismique", dont le déplacement relatif par rapport à un transducteur piézoélectrique, électromagnétique, ou électrostatique génère une grandeur électrique. D'autres méthodes de récupération d'énergie pour alimenter des implants cardiaques ont été proposées, comme l'utilisation en régime quasi-statique des déplacements du corps ou des organes. C'est le cas des systèmes du type montre à remontage automatique, qui restent cependant trop volumineux et ne fournissent pas assez d'énergie, ou du cas spécifique des générateurs piézoélectriques, fournissant une grande puissance mais aux dimensions macroscopiques. D'autres méthodes nécessitant plusieurs points d'ancrage ont été proposées, mais sont difficiles à implémenter et ont un caractère invasif et facilement inflammatoire.

Les US 7 729 768, US 3 456 134 et US 2010/0217364 proposent une configuration de récupérateur inertiel dans laquelle une masse sismique oscillante et des moyens de récupération d'énergie associés sont disposés dans une chambre, à l'intérieur d'un boîtier de capsule *leadless.* Cette masse peut être entraînée selon au moins un degré de liberté par le mouvement global de la capsule, de ce qui permet à des moyens de récupération de convertir en énergie électrique un déplacement relatif de la masse sismique par rapport au boîtier. La quantité d'énergie convertie est proportionnelle à la masse et donc au volume de la masse sismique, et est directement liée à l'amplitude de l'accélération des vibrations à laquelle cette masse est soumise. La capsule est en contact direct avec les tissus cardiaques par l'intermédiaire d'une embase rigide afin d'assurer les fonctions de stimulation et de détection des signaux de dépolarisation cardiaque. Ainsi, l'accélération subie par la masse sismique est l'accélération de la paroi cardiaque à laquelle la capsule est attachée par des moyens d'ancrage situés sur l'embase.

Le US 2007/0293904 A1 décrit encore un autre dispositif de récupération d'énergie, où les variations de la pression sanguine déforment un boîtier en forme de soufflet ancré à la paroi cardiaque, avec mise en résonance d'une masse oscillante pilotant un générateur piézoélectrique ou électromagnétique.

Le point de départ de l'invention est la constatation du fait que les capsules endocavitaires sont non seulement soumises au mouvement de la paroi de l'organe du patient dans laquelle elles sont ancrées, mais aussi aux forces et turbulences des fluides dans lesquels elles baignent. On peut de ce fait envisager d'augmenter l'efficacité de conversion des capsules en récupérant également l'énergie mécanique développée par les efforts fluidiques, qui participent à leur manière au mouvement relatif de la masse sismique par rapport au boîtier et aux moyens de récupération. Cependant, une telle récupération supplémentaire ne doit pas avoir pour conséquence d'augmenter la masse ni le volume de la masse sismique, ceci pour des raisons de respect de la miniaturisation.

Aussi, un but de l'invention est de proposer une capsule du type *leadless* qui permettrait de transmettre simultanément à la masse sismique les mouvements des parois d'organes et des flux fluidiques sans en augmenter la masse ni le volume.

Ce but est atteint, conformément à l'invention, grâce à une capsule médicale intracorporelle autonome comportant, comme divulgué par exemple par le US 2007/0293904 précité, un boîtier définissant une chambre dans laquelle sont disposés une masse sismique oscillante et des moyens de récupération d'énergie aptes à convertir en énergie électrique un déplacement relatif de la masse sismique par rapport au boîtier, et, d'autre part, des moyens d'ancrage du boîtier à une paroi d'un organe d'un patient. De façon caractéristique de l'invention, la capsule comprend en outre une embase élastiquement déformable reliée au boîtier à une première extrémité et portant les moyens d'ancrage à une deuxième extrémité.

Du point de vue dynamique, on comprend que la masse sismique de la capsule selon l'invention est soumise, comme pour les capsules connues de l'état de la technique, aux efforts dus aux mouvements de la paroi d'organe qui lui sont transmis par l'embase. Selon l'invention, la masse sismique est soumise en outre aux forces fluidiques appliquées sur la capsule, ceci du fait que l'élasticité de l'embase permet au boîtier de se déplacer par rapport à la paroi. Dans l'état de la technique, cette composante fluidique est inopérante car l'embase est alors rigide et limite considérablement les mouvements du boîtier.

Il est donc possible de récupérer à la fois l'énergie mécanique des mouvements des parois d'organes et celle fournie par les flux fluidiques, sans qu'il soit nécessaire d'augmenter la masse ni le volume de la masse sismique.

Par ailleurs, il faut souligner un autre avantage de l'invention concernant l'ancrage de la capsule. En effet, dans l'état de la technique, la capsule est fixée à la paroi par une embase rigide, ce qui a pour conséquence que les forces et turbulences du flux sanguin exercés sur la capsule sont mécaniquement transmises au point d'ancrage dans la paroi cardiaque et ont tendance à le fragiliser avec le risque d'arrachement de la capsule. Au contraire, avec l'invention, ce risque est minimisé par l'élasticité de l'embase, apte alors à absorber les efforts fluidiques appliqués sans les transmettre aux moyens d'ancrage.

Comme on le verra en détail plus bas, on peut modéliser le système inertiel constitué par la capsule selon l'invention par deux oscillateurs couplés, l'un formé par la masse sismique oscillant dans le boîtier, et l'autre par le boîtier lui-même oscillant par rapport à la paroi d'organe, à l'extrémité de l'embase élastiquement déformable formant ressort.

Le spectre du déplacement relatif de la masse sismique présente une bande de fréquence élargie conduisant à une récupération la plus efficace. Selon l'invention, les masses et les coefficients de forces de rappel de la masse sismique et du boîtier sont choisis de sorte que ce spectre du déplacement relatif de la masse sismique par rapport au boîtier présente un premier maximum entre 0,5 et 10 Hz et un deuxième maximum entre 12 et 40 Hz.

Cette disposition avantageuse a pour effet de faire coïncider les fréquences des maxima du spectre du déplacement relatif de la masse sismique avec des intervalles de fréquence typiques de pulsation du flux sanguin (0,5 à 10 Hz) et de mouvement des parois cardiaques (15 à 40 Hz).

Plus particulièrement, le premier maximum est situé à 1,2 Hz et le deuxième maximum est situé à 18 Hz.

Selon un premier mode de réalisation, la masse sismique et l'embase sont configurées de sorte que le mouvement d'oscillation de la masse sismique et le mouvement de déformation de l'embase s'effectuent selon des directions principales longitudinales, orientées perpendiculairement à la paroi de l'organe du patient. Selon un deuxième mode de réalisation, la masse sismique et l'embase sont configurées de sorte que le mouvement d'oscillation de la masse sismique et le mouvement de déformation de l'embase s'effectuent selon des directions principales transversales, orientées parallèlement à la paroi de l'organe du patient.

Avantageusement, la capsule de l'invention comprend des moyens d'oscillation de la masse sismique constitués par une poutre encastrée, un ressort gravé ou un ressort spiralé et des moyens de déformation élastique de l'embase constitués par un ressort hélicoïdal ou une poutre encastrée.

Enfin, la capsule selon l'invention peut comprendre des moyens de fixation réversible du boîtier sur l'embase. Il est ainsi possible de remplacer la capsule facilement sans détacher les moyens d'ancrage des tissus cardiaques.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue de côté d'une capsule à excitation longitudinale conforme à l'invention, placée dans une cavité cardiaque.

La Figure 2 est un schéma en vue de côté de la capsule de la Figure 1.

La Figure 3 est un schéma dynamique équivalent de la capsule des Figures 1 et 2.

La Figure 4 représente un diagramme de Bode du rapport en dB du déplacement $U$ de la masse sismique de la Figure 3 au déplacement $u_g$ de la paroi cardiaque en fonction de la pulsation normalisée $\omega_c$, pour différentes valeurs du paramètre $\alpha$.

La Figure 5 représente un diagramme de Bode du rapport en dB du déplacement $U$ de la masse sismique de la Figure 3 au déplacement dû au fluide $F/k$ en fonction de la pulsation normalisée $\omega_{\overline{c}}$, pour différentes valeurs du paramètre $\alpha$.

La Figure 6 représente un diagramme de Bode du rapport en dB du déplacement $U$ de la masse sismique de la Figure 3 au déplacement $u_g$ de la paroi cardiaque ou au déplacement dû au fluide $F/k$ en fonction de la pulsation normalisée $\omega_c$, pour différentes valeurs du paramètre $\beta$ et pour $\alpha = 1$.

La Figure 7a est une vue de côté d'un premier mode de réalisation d'une capsule conforme à l'invention.

La Figure 7b est une vue de côté d'un deuxième mode de réalisation d'une capsule conforme à l'invention.

La Figure 8a est un schéma en vue de côté d'un premier mode de réalisation d'une capsule à excitation latérale conforme à l'invention.

La Figure 8b est un schéma en vue de côté d'un deuxième mode de réalisation d'une capsule à excitation latérale conforme à l'invention.

La Figure 9a est une vue en perspective d'un premier mode de réalisation de moyens d'oscillation de la masse sismique.

La Figure 9b est une vue en perspective d'un deuxième mode de réalisation de moyens d'oscillation de la masse sismique.

La Figure 9c est une vue en perspective d'un troisième mode de réalisation de moyens d'oscillation de la masse sismique.

La Figure 10a est une vue en perspective d'un premier mode de réalisation de moyens de déformation élastique de l'embase.

La Figure 10b est une vue en perspective d'un deuxième mode de réalisation de moyens de déformation élastique de l'embase.

La Figure 10c est une vue en perspective d'un troisième mode de réalisation de moyens de déformation élastique de l'embase.

On va maintenant décrire et expliquer divers exemples de mise en oeuvre de l'invention.

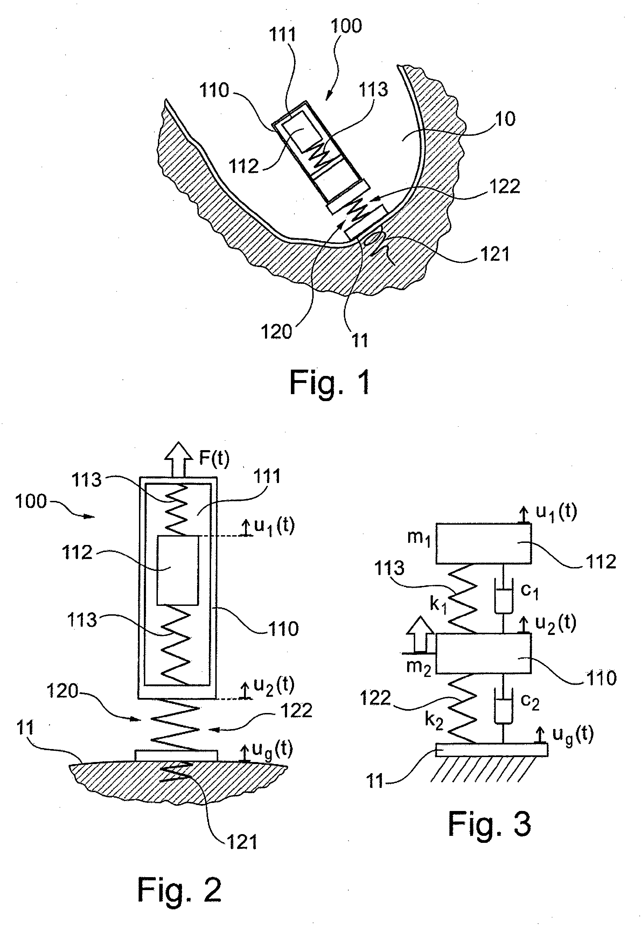

Sur la Figure 1 est représentée une capsule médicale intracorporelle autonome 100 du type *leadless,* comportant un boîtier 110 et une embase 120 portant des moyens 121 d'ancrage destinés à fixer la capsule à une paroi 11 d'une cavité cardiaque 10 d'un patient. Dans l'exemple de la Figure 1, les moyens 121 sont des moyens à vis d'ancrage.

Sur le plan fonctionnel, la capsule 100 comprend de nombreux éléments qui ne sont pas représentés sur la Figure 1, tels qu'une électrode (qui peut être incorporée à la vis 121 d'ancrage) pour transmettre à des circuits électroniques appropriés des potentiels de dépolarisation du myocarde ou pour appliquer des impulsions de stimulation à la paroi cardiaque 11. D'autres capteurs peuvent être incorporés à la capsule 100, comme des capteurs de niveau d'oxygène dans le sang, de pression cardiaque, etc. ainsi que des moyens émetteurs/récepteurs de communication sans fil. L'autonomie énergétique de la capsule 100 est garantie du fait qu'elle comporte un système de récupération d'énergie basé sur un dispositif inertiel qui, dans son principe, met à profit l'accélération subie de la part du milieu dans lequel se trouve la capsule, pour déplacer une masse sismique oscillante 112 sous l'action de rappel d'un ressort 113 à l'intérieur d'une chambre 111 du boîtier 110. Le mouvement relatif de la masse sismique par rapport au boîtier est converti en énergie électrique par des moyens de récupération (non représentés) disposés dans la chambre 111. Ces moyens de récupération peuvent être, de manière connue, des moyens de transduction piézoélectrique, électromagnétique ou électrostatique.

Comme on peut le voir sur la Figure 1, l'embase 120 est constituée d'un corps élastiquement déformable 122 relié au boîtier 110 à une première extrémité et portant les moyens 121 d'ancrage à une deuxième extrémité. Sur cette figure, le corps élastiquement déformable est représenté par un ressort 122.

Cette caractéristique présente par rapport aux dispositifs inertiels connus de l'état de la technique l'avantage de permettre une récupération d'énergie à double effet, à savoir la récupération (i) des mouvements de la paroi cardiaque ainsi que (ii) des forces fluidiques à l'intérieur de la cavité 10. On rappellera à cet égard que, dans l'état de la technique, la récupération ne porte que sur les mouvements de la paroi, l'embase étant rigide et non élastiquement déformable.

La Figure 2 est un schéma de la capsule 100 en mode d'excitation fluidique longitudinale, au sens où la masse sismique 112 et l'embase 120 sont configurées de sorte que le mouvement d'oscillation de la masse sismique et le mouvement de déformation de l'embase s'effectuent longitudinalement dans une direction sensiblement normale à la paroi 11 correspondant à l'axe de la capsule 100.

La Figure 3 est une modélisation dynamique de la capsule de la Figure 2 : $m_1$ est la masse sismique 112, $k_1$ la raideur du ressort associé 113 et $C_1$ la constante de frottement visqueux, $m_2$ est la masse du boitier 110 et des parties fixes de la capsule (électronique, capteurs, etc.), $k_2$ la raideur du ressort 122 équivalent à l'embase 120 et $C_2$ les pertes mécaniques de cette dernière.

Par ailleurs, $u_g$, $u_1$ et $u_2$ sont les déplacements respectifs de la paroi cardiaque 11, du boîtier 110 et de la masse sismique 112. Enfin, $F$ est la force d'excitation longitudinale due aux efforts développés par fluide environnant sur la capsule 100.

On remarquera que le coefficient $C_1$ représente à la fois le couplage de transduction et les pertes mécaniques sous forme visqueuse. Ces dernières sont très faibles devant l'amortissement de transduction et peuvent être négligées. Pour cette même raison, le coefficient $C_2$ peut être négligé devant $C_1$.

L'équation fondamentale de la dynamique appliquée au système de la Figure 3 s'écrit sous forme matricielle de la manière suivante :

$$\begin{pmatrix} m_1 & 0 \\ 0 & m_2 \end{pmatrix} \begin{pmatrix} \ddot{u}_1 \\ \ddot{u}_2 \end{pmatrix} + \begin{pmatrix} c_1 & -c_1 \\ -c_1 & c_1 + c_2 \end{pmatrix} \begin{pmatrix} \dot{u}_1 \\ \dot{u}_2 \end{pmatrix} + \begin{pmatrix} k_1 & -k_1 \\ -k_1 & k_1 + k_2 \end{pmatrix} \begin{pmatrix} u_1 \\ u_2 \end{pmatrix} = \begin{pmatrix} 0 \\ k_2 u_g + c_2 \dot{u}_g + F \end{pmatrix} = P$$

Dans cette équation les efforts sont découplés par linéarité en deux composantes $P_1$, $P_2$ :

$$P = \begin{pmatrix} 0 \\ P_1 + P_2 \end{pmatrix}$$

où $P_1 = k_2 u_g + c_2 u_g$ représente les efforts dus aux mouvements sismiques de la paroi, et $P_2 = F$ , $F$ correspondant aux forces fluidiques appliquées sur la capsule 100 dans la cavité 10.

En supposant la charge $P_i$ *(i = 1,2)* harmonique à la pulsation $\omega$ et en passant en représentation complexe, l'amplitude $U = U_1 - U_2$ de la position relative de la masse sismique 112 est donnée (en négligeant $C_2$ devant $C_1$) par :

$$U = \frac{P_i \omega_c^2 / k}{\alpha + 2i\alpha\xi\omega_c - (1 + \alpha + \beta)\omega_c^2 - 2i(1+\beta)\xi\omega_c^3 + \beta\omega_c^4}$$

Dans cette expression, les changements de notation suivants ont été effectués :

$$k_1 = k, \ k_2 = \alpha k_1 = \alpha k \ ,$$

$$m_1 = m, \ m_2 = \beta m_1 = \beta m,$$

$$c_1 = c, \ \omega_n = \sqrt{k/m},$$

$$\omega_c = \omega / \omega_n,$$

$$\xi = c_1/(2\sqrt{km})$$

La pulsation normalisée $\omega_c$ est donc la pulsation w des efforts appliqués ramenée à la pulsation propre $\omega_n$ de la masse sismique oscillante 112. On peut déduire de la formule précédente l'expression de $U$ pour chacune des sources d'énergie, à savoir :

- pour les mouvements cardiaques : $P_1 = k_2 u_g = \alpha k u_g$, d'où :

$$U = \frac{\alpha\omega_c^2}{\alpha + 2i\alpha\xi\omega_c - (1 + \alpha + \beta)\omega_c^2 - 2i(1+\beta)\xi\omega_c^3 + \beta\omega_c^4} u_g$$

- pour les forces fluidiques : $P_2 = F$ , d'où :

$$U = \frac{\omega_c^2}{\alpha + 2i\alpha\xi\omega_c - (1 + \alpha + \beta)\omega_c^2 - 2i(1+\beta)\xi\omega_c^3 + \beta\omega_c^4} \frac{F}{k}$$

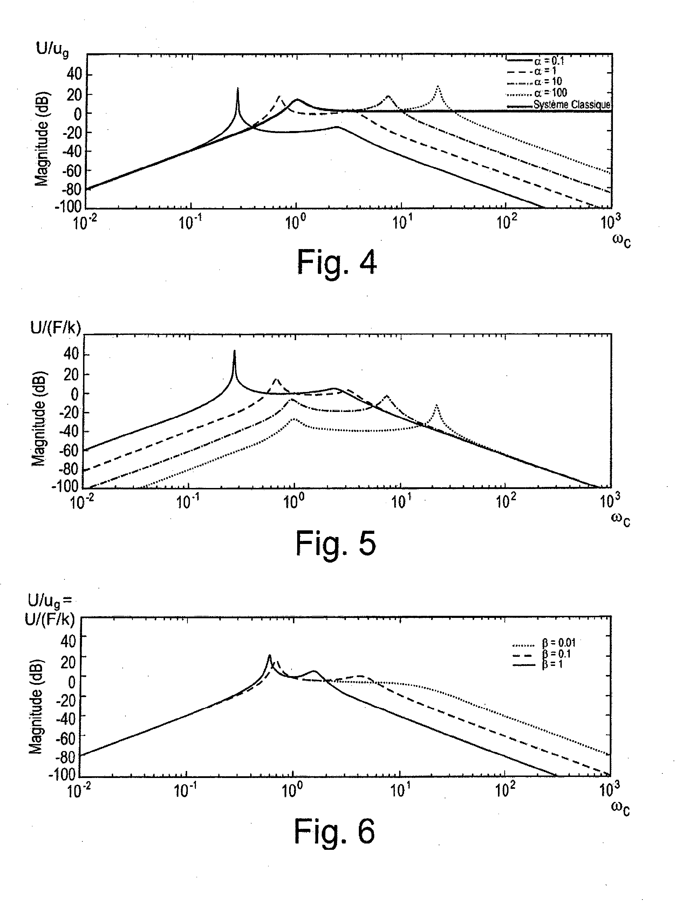

Les Figures 4 et 5 sont les diagrammes de Bode montrant comment varient (en dB), respectivement les rapports $U/u_g$ et $U/(F/k)$, en fonction de la fréquence normalisée $\omega_c$, ceci pour différentes valeurs du paramètre $\alpha = k_2/k_1$.

On peut observer que ces diagrammes présentent une bande élargie dans laquelle la récupération est effectuée avec une bien meilleure efficacité que pour les dispositifs inertiels connus de l'état de la technique. D'autre part, ils font apparaître en général deux maxima, ou pics, pour lesquels la récupération est optimale. Ces pics correspondent à des résonances à des pseudo-fréquences propres du système mécanique masse sismique/boîtier à deux degrés de liberté d'oscillation.

Le comportement de ces courbes en fonction du paramètre $\alpha$ peut être interprété de la manière suivante.

S'agissant du comportement horizontal, on constate que lorsque $\alpha$ est petit, les deux pics se déplacent vers la gauche pour les deux courbes. Le système est globalement plus flexible. Quand $\alpha$ est grand, le premier pic tend vers

$\omega_c$ = 1, le deuxième pic s'éloigne pour les deux courbes. Dans le cas limite où $\alpha = \infty$, il n'y a qu'un seul pic à $\omega_c$ = 1. On se alors trouve dans la situation classique de l'état de la technique où $k_2 = \infty$ et pour laquelle seuls les mouvements de paroi cardiaque sont récupérés à la fréquence de résonance $\omega_n$ de la masse sismique.

S'agissant du comportement horizontal, on peut voir que quand $\alpha$ diminue, le niveau du plateau moyen de la courbe $U/u_g$ descend alors que celui de la courbe $U/(F/k)$ tend vers 1. On récupère alors plus de forces fluidiques que de mouvements de paroi. Quand $\alpha$ augmente, c'est l'inverse : le niveau du plateau moyen de la courbe $U/ug$ tend vers 1 tandis que celui de la courbe $U/(F/k)$ diminue.

En conclusion, si l'on veut récupérer de manière égale les mouvements de paroi et les efforts fluidiques le choix $\alpha$ = 1 est un bon compromis.

La Figure 6 représente un diagramme de Bode donnant pour $\alpha$ = 1 les variations en dB de $U/u_g = U/(F/k)$ en fonction de la fréquence normalisée $\omega_c$, pour différentes valeurs du paramètre $\beta = m_2/m_1$.

D'une manière générale, si $\beta$ augmente, les pics se rapprochent, se décalent vers la gauche et s'amplifient. Il est donc préférable d'augmenter $\beta$ pour avoir de plus grands pics de résonance tout en cherchant à faire coïncider les pics avec les spectres d'excitation situés entre 0,5 et 10 Hz pour les flux sanguins, et entre 15 et 40 Hz pour les mouvements cardiaques, les fréquences préférées étant respectivement de 1,2 Hz et 18 Hz.

Pour fixer quelques ordres de grandeur, on peut supposer que la paroi cardiaque varie avec une amplitude $u_g$ de quelques millimètres lors du battement.

On peut également assimiler la force $F$ du fluide, notamment dans le cas d'une force perpendiculaire au dispositif, par la traînée appliquée par le flux sanguin. Dans cette hypothèse, on peut quantifier l'amplitude de cette force variant périodiquement selon le cycle cardiaque par la formule :

$$F = \frac{1}{2} C \rho S V^2$$

où $C$ est le coefficient de trainée (qui vaut environ 0.5 pour un cylindre tel que le dispositif illustré sur les figures), $\rho$ est la masse volumique du sang (environ 1000 kg/m$^3$), $S$ est la surface efficace en regard du fluide (de l'ordre de 50 mm$^2$), et $V$ est la vitesse du sang (environ 1 m/s pour le haut de l'intervalle de variation). Ainsi, on peut prendre suivant cet exemple de géométrie une force $F$ ayant un ordre de grandeur d'une dizaine de mN.

La masse $m_2$ est due au boîtier et à l'électronique, soit un volume d'environ 150 mm$^3$ qui se décompose en 100 mm$^3$ pour l'électronique et 50 mm$^3$ pour le boîtier, ce qui représente quelques dixièmes de grammes. La masse sismique $m_1$, qui doit être conçue pour être importante et donc dans un matériau très dense tel que le tungstène, pèse d'un à quelques grammes. Ainsi, la valeur de $\beta = m_2/m_1$ se situe autour de quelques dixièmes, typiquement 0,2.

La valeur de $\alpha$, déterminée par la forme et la nature de la raideur $k_2$, peut être ajustée, notamment autour de 1, lors de la conception du récupérateur. La valeur de $\xi$, donnant l'amortissement, est choisie à environ 0,1, qui est l'ordre de grandeur donnant en général le meilleur compromis bande passante/puissance/déplacement.

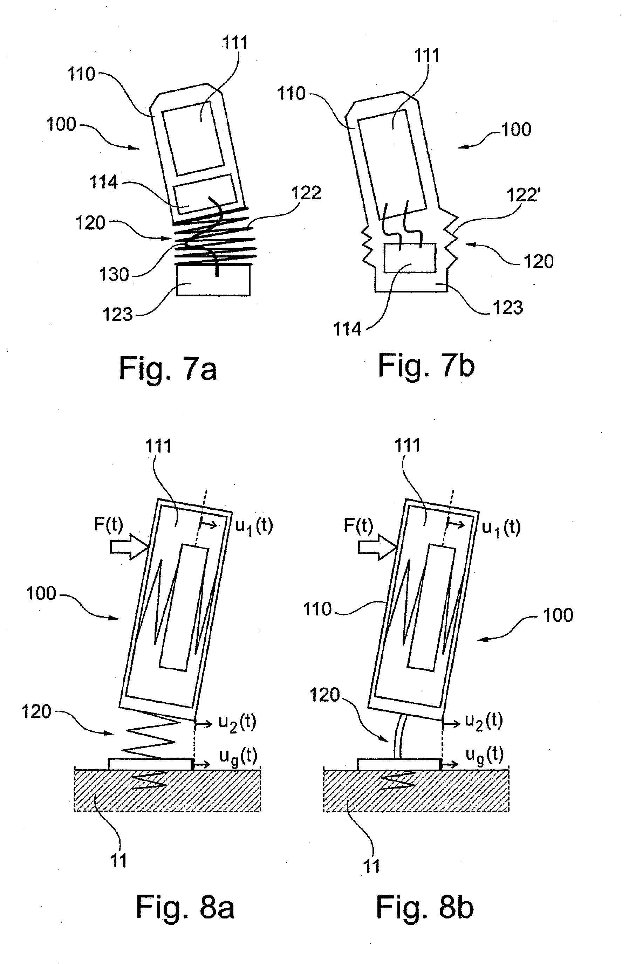

La Figure 7a illustre un premier mode de réalisation de la capsule 100 où la chambre 111 de récupération d'énergie, comprenant la masse sismique et les moyens de récupération d'énergie, ainsi que le module électronique 114 sont positionnés dans le boîtier 110, lequel constitue la partie mobile de la capsule. La semelle fixe 123 située à une extrémité de l'embase 120 permet l'ancrage de la capsule 100 dans les tissus cardiaques par les moyens à vis précédemment décrits.

L'élément flexible 122 assure le couplage mécanique entre le boîtier de la capsule et le mouvement de la paroi cardiaque subi par la semelle 123. Cet élément flexible peut être réalisé à l'aide d'un ressort métallique. Seul le boîtier mobile 110 doit être étanche dans cette configuration. Un conducteur souple 130 permet d'interfacer le module électronique avec une ou plusieurs électrodes situées dans la semelle 123.

Selon le deuxième mode de réalisation montré sur la Figure 7b, le module électronique 114 et la chambre 111 de récupération d'énergie sont positionnés dans un tube articulé. L'articulation flexible peut être réalisée par exemple par un soufflet 122'. Dans ce cas de figure, l'étanchéité doit être assurée au niveau de l'interface entre le tube et les électrodes en extrémité de la semelle 123.

En outre, le boîtier 110 et l'embase 120 peuvent être désolidarisés grâce à des moyens de fixation réversible (non représentés) dans le but d'améliorer la modularité de la capsule et d'en faciliter éventuellement l'explantation.

Concernant la réalisation et le dimensionnement du ressort interne 113 de la masse oscillante 112 et du ressort externe 122 de l'embase 120, il convient de distinguer deux modes de fonctionnement principaux, à savoir le mode de fonctionnement longitudinal illustré à la Figure 2 et le fonctionnement latéral illustré aux Figures 8a et 8b, en fonction de la direction d'excitation que l'on souhaite privilégier : perpendiculairement ou parallèlement à la paroi cardiaque 11.

Supposons que l'on souhaite concevoir un dispositif où $\alpha$ vaut 1 (selon un exemple de compromis trouvé des diagrammes de Bode ci-dessus) et où l'on souhaite avoir $\omega_c$ égal à environ 0,5, correspondant au pic le plus important des courbes. Prenons le cas d'une excitation à environ $\omega$ = 20 Hz, c'est-à-dire une forte composante d'un battement cardiaque typique. On a alors : $\omega_n = \omega/\omega_c = 2.2\pi.20 \approx 250$ rad/s, soit une raideur $k \approx 250$ N/m avec $m_1 \approx 4$ g.

Pour le dispositif oscillant, la masse sismique 112 d'environ 4 g peut être avantageusement obtenue en utilisant un matériau très dense tel que le tungstène, avec par exemple des dimensions de 8 x 5 x 5 mm$^3$ environ.

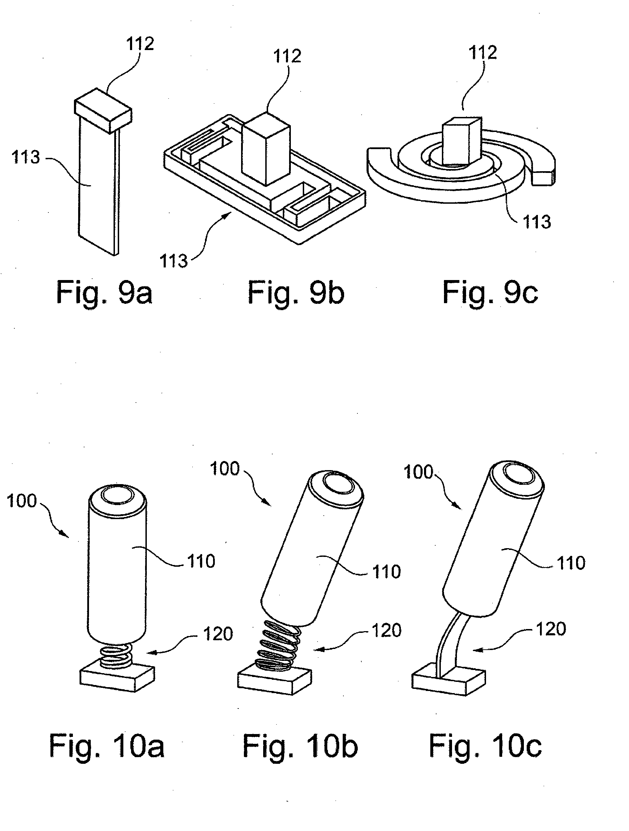

La manière la plus simple de concevoir le ressort interne 113, notamment si l'on souhaite récupérer les efforts latéraux conformément aux Figures 8a ou 8b, est de réaliser une simple poutre comme celle illustrée sur la Figure 9a.

Pour obtenir le coefficient de raideur $k_1 = k = 250$ N/m, cette poutre peut avoir les propriétés géométriques suivantes : module d'Young 150 GPa (ordre de grandeur pour beaucoup de métaux), largeur 5 mm, épaisseur 75 $\mu$m, longueur 7,5 mm. La raideur peut être déterminée par la formule issue de la théorie d'Euler-Bernoulli pour une poutre encastrée-libre : $k = E.w.(t/L)^{3}/4$, où $E$ est le module d'Young du matériau, $t$ est l'épaisseur de la poutre, $w$ sa largeur et $L$ sa longueur.

Le ressort interne peut aussi être microstructuré dans du silicium par gravure profonde d'une plaque, selon le dispositif de la Figure 9b.

Pour obtenir la raideur souhaitée, on utilise une autre formule issue de la théorie des poutres pour de tels ressorts : $k = 2.E.w.(t/L)^{3/n}$ où $E$ est le module d'Young du matériau (environ 150 GPa pour le silicium), $w$ est l'épaisseur des ressorts, $t$ leur largeur, $L$ la longueur de chaque bras et $n$ le nombre de bras composant chaque ressort, soit $n$ = 2 dans le cas de la Figure 9b. Pour obtenir $k_1$ = 250 N/m, on peut par exemple structurer dans une plaque de silicium classique des ressorts de 500 $\mu$m de hauteur, 75 $\mu$m d'épaisseur et 5 mm de longueur. Selon l'orientation que l'on donne au dispositif, ce ressort peut être utilisé en configuration latérale ou longitudinale, pourvu que la masse collée au ressort ait un facteur de forme compatible avec le conditionnement global. Il faut toutefois remarquer que cette disposition se prête plus à l'excitation longitudinale que latérale.

Enfin, on peut également envisager un ressort interne pour masse sismique conçu selon une spirale, comme représenté sur la Figure 9c.

Ce cas est particulièrement adapté à la récupération longitudinale. De manière similaire aux cas précédents, on peut déterminer les propriétés de cette spirale, par exemple : diamètre 4 mm, deux bras faisant un tour chacun, ayant une longueur d'environ 10 mm, une largeur de 500 $\mu$m et une épaisseur de 250 $\mu$m. Pour le ressort externe, dans le cas détaillé précédemment où $\alpha$ vaut 1, la raideur $k_2$ doit également être de 250 N/m environ.

Pour une excitation longitudinale, on peut par exemple prendre un ressort de type hélicoïdal tel qu'illustré sur la Figure 10a.

La raideur de ce ressort est donnée par : $k = E.d^4/(16.(1+v)(D-d)^3.n)$, en fonction du diamètre $d$ du fil, de celui $D$ du ressort, du module d'Young $E$, du coefficient de Poisson $v$ et du nombre de spires $n$. Le ressort peut ainsi être fait de métal, par exemple de titane, et présenter un diamètre extérieur de 3 mm et un diamètre de fil de 250 $\mu$m, et avoir $n$ = 5 spires.

Pour une excitation latérale, on peut utiliser un même ressort hélicoïdal, mais sollicité en flexion, conformément à la Figure 10b.

Une simulation numérique a montré qu'une raideur de 250 N/m pouvait être obtenue avec un dimensionnement, relativement voisin du précédent, où le ressort a un diamètre extérieur de 3 mm, un nombre de spires égal à 5 et un diamètre de fil de 375 $\mu$m.

On peut également, en excitation latérale, utiliser un ressort du type poutre montré sur la Figure 10c, similaire à la version du ressort interne de la Figure 9a.

Comparé à ce dernier, il y a avantage à ce qu'il soit constitué en matière plus souple comme un polymère, si l'on souhaite qu'il ne soit pas trop fin ni trop long. Par exemple, il peut être réalisé en PEEK (*PolyEther Ether Ketone*), avoir une longueur de 4 mm, une épaisseur de 200 pm et une largeur de 3 mm.

Il est à noter que les éléments de ressort peuvent être combinés pour récupérer les efforts suivant plusieurs directions à la fois, pourvu que le mouvement final de la masse sismique puisse être converti en électricité par le transducteur.

Une fois qu'un déplacement relatif variable $U$ est obtenu entre la masse sismique 112 et le boitier 110, plusieurs moyens de transduction peuvent être appliqués :

- moyens électromagnétiques, comme ceux décrits par exemple dans le US 2010/0176664 : un moyen crée un champ magnétique, par exemple avec un aimant permanent (lequel peut d'ailleurs constituer la masse sismique), tandis qu'un autre moyen comprend un circuit en mouvement relatif par rapport au premier et dans lequel un courant circule par induction ;

- moyens électrostatiques : chacun des moyens possède un jeu d'électrodes en regard les unes des autres, créant ainsi un condensateur. Lors d'un mouvement relatif, la capacité de ce condensateur varie, soit en changeant l'entrefer, soit en changeant les surfaces en regard. Cette variation de capacité peut être convertie en énergie en préchargeant le condensateur, de manière externe ou par l'intermédiaire d'électrets, lorsque la capacité est élevée et en le déchargeant lorsque la capacité est faible ;

- moyens piézoélectriques : le ressort est réalisé au moins en partie en un matériau piézoélectrique et disposé entre les deux moyens, et il est de ce fait soumis à des déformations lors d'un mouvement relatif. Lorsqu'il se déforme, des charges électriques sont créées directement par piézoélectricité sur des électrodes placées aux extrémités de l'élément piézoélectrique.

**Revendications**

1. Une capsule (100) médicale intracorporelle autonome comportant :

   - un boîtier (110) définissant une chambre (111) dans laquelle sont disposés une masse sismique oscillante (112) et des moyens de récupération d'énergie aptes à convertir en énergie électrique un déplacement relatif ($U$) de la masse sismique (112) par rapport au boîtier ; et
   - des moyens (121) d'ancrage du boîtier (110) à une paroi (11) d'un organe d'un patient,

   **caractérisée en ce que** la capsule (100) comprend en outre :

   - une embase élastiquement déformable (120), reliée au boîtier (110) à une première extrémité et portant les moyens (121) d'ancrage à une deuxième extrémité.

2. La capsule de la revendication 1, dans laquelle les masses ($m_1$, $m_2$) et les coefficients ($k_1$, $k_2$) des forces de rappel de la masse sismique oscillante (112) et du boîtier (110) sont choisis de sorte que le spectre du déplacement relatif ($U$) de la masse sismique par rapport au boîtier présente un premier maximum entre 0,5 et 10 Hz et un deuxième maximum entre 15 et 40 Hz.

3. La capsule selon la revendication 2, dans laquelle le premier maximum est situé à 1,2 Hz.

4. La capsule selon la revendication 2, dans laquelle le deuxième maximum est situé à 18 Hz.

5. La capsule selon la revendication 1, dans laquelle les coefficients ($k_1$, $k_2$) des forces de rappel de la masse sismique oscillante (112) et du boîtier (110) sont dans un rapport ($\alpha$) égal à 1.

6. La capsule de la revendication 1, dans laquelle la masse sismique (112) et l'embase (120) sont configurées de sorte que le mouvement d'oscillation de la masse sismique et le mouvement de déformation de l'embase s'effectuent selon des directions principales longitudinales, orientées perpendiculairement à la paroi (11) de l'organe du patient.

7. La capsule de la revendication 1, dans laquelle la masse sismique (112) et l'embase (120) sont configurées de sorte que le mouvement d'oscillation de la masse sismique et le mouvement de déformation de l'embase s'effectuent selon des directions principales transversales, orientées parallèlement à la paroi (11) de l'organe du patient.

8. La capsule de la revendication 1, comprenant des moyens (113) d'oscillation de la masse sismique (112) constitués par une poutre encastrée, un ressort gravé ou un ressort spiralé.

9. La capsule de la revendication 1, comprenant des moyens (122) de déformation élastique de l'embase (120) constitués par un ressort hélicoïdal ou une poutre encastrée.

10. La capsule de la revendication 1, comprenant des moyens de fixation réversible du boîtier sur l'embase.

**Patentansprüche**

1. Medizinische körperinterne autonome Kapsel (100), die Folgendes aufweist:

   - ein Gehäuse (110), das eine Kammer (111) definiert, in der eine oszillierende seismische Masse (112) und Energierückgewinnungsmittel angeordnet sind, die geeignet sind, um eine relative Bewegung (U) der seismischen Masse (112) in Bezug auf das Gehäuse in elektrische Energie umzuwandeln, und
   - Mittel (121) zum Verankern des Gehäuses (110) an einer Wand (11) eines Organs eines Patienten,

**dadurch gekennzeichnet, dass** die Kapsel (100) ferner Folgendes aufweist:

- eine elastisch verformbare Basis (120), die mit dem Gehäuse (110) mit einem ersten Ende verbunden ist und Mittel (121) zum Verankern an einem zweiten Ende aufweist.

2. Kapsel nach Anspruch 1, bei der die Massen ($m_1$, $m_2$) und die Koeffizienten ($k_1$, $k_2$) der Rückholkräfte der oszillierenden seismischen Masse (112) und des Gehäuses (110) derart ausgewählt sind, dass das Spektrum der relativen Bewegung (U) der seismischen Masse in Bezug auf das Gehäuse ein erstes Maximum zwischen 0,5 und 10 Hz und ein zweites Maximum zwischen 15 und 40 Hz aufweist.

3. Kapsel nach Anspruch 2, bei der das erste Maximum bei 1,2 Hz liegt.

4. Kapsel nach Anspruch 2, bei der das zweite Maximum bei 18 Hz liegt.

5. Kapsel nach Anspruch 1, bei der die Koeffizienten ($k_1$, $k_2$) der Rückholkräfte der oszillierenden seismischen Masse (112) und des Gehäuses (110) in einem Verhältnis ($\alpha$) gleich 1 sind.

6. Kapsel nach Anspruch 1, bei der die seismische Masse (112) und der Sockel (120) derart konfiguriert sind, dass die Oszillationsbewegung der seismischen Masse und die Verformungsbewegung des Sockels entlang Hauptlängsrichtungen, die senkrecht zu der Wand (11) des Organs des Patienten ausgerichtet sind, erfolgen.

7. Kapsel nach Anspruch 1, bei der die seismische Masse (112) und der Sockel (120) derart konfiguriert sind, dass die Oszillationsbewegung der seismischen Masse und die Verformungsbewegung des Sockels entlang Hauptquerrichtungen, die parallel zu der Wand (11) des Organs des Patienten ausgerichtet sind, erfolgen.

8. Kapsel nach Anspruch 1, die Mittel (113) zum Oszillieren der seismischen Masse (112) aufweisen, die aus einem eingelassenen Balken, einer gravierten Feder oder einer Schlossfeder bestehen.

9. Kapsel nach Anspruch 1, die Mittel (122) zur elastischen Verformung des Sockels (120), die aus einer Spiralfeder oder einem eingelassenen Balken bestehen, aufweist.

10. Kapsel nach Anspruch 1, die umkehrbare Befestigungsmittel des Gehäuses auf dem Sockel aufweist.

**Claims**

1. An autonomous intracorporeal medical capsule (100) including:

- a housing (110) defining a chamber (111) in which are arranged an oscillating seismic mass (112) and energy-harvesting means adapted to convert into electrical energy a relative displacement (U) of the seismic mass (112) with respect to the housing ; and
- means (121) for the anchoring of the housing (110) to a wall (11) of an organ of a patient,
**characterized in that** the capsule (100) further comprises:
- an elastically deformable base (120), connected to the housing (110) at a first end and bearing the anchoring means (121) at a second end.

2. The capsule of claim 1, wherein the masses ($m_1$, $m_2$) and the coefficients ($k_1$, $k_2$) of the return forces of the oscillating seismic mass (112) and of the housing (110) are chosen so that the spectrum of the relative displacement (U) of the seismic mass with respect to the housing has a first maximum between 0.5 and 10 Hz and a second maximum between 15 and 40 Hz.

3. The capsule of claim 2, wherein the first maximum is located at 1.2 Hz.

4. The capsule of claim 2, wherein the second maximum is located at 18 Hz.

5. The capsule of claim 1, wherein the coefficients ($k_1$, $k_2$) of the return forces of the oscillating seismic mass (112) and of the housing (110) are in a ratio ($\alpha$) equal to 1.

6. The capsule of claim 1, wherein the seismic mass (112) and the base (120) are configured so that the oscillating movement of the seismic mass and the deformation movement of the base are along longitudinal main directions, oriented perpendicular to the wall (11) of the organ of the patient.

7. The capsule of claim 1, wherein the seismic mass (112) and the base (120) are configured so that the oscillating movement of the seismic mass and the deformation movement of the base are along transverse main directions, oriented parallel to the wall (11) of the organ of the patient.

8. The capsule of claim 1, comprising means (113) for the oscillation of the seismic mass (112) consisted of a fixed beam, an etched spring or a spiralled spring.

9. The capsule of claim 1, comprising means (122) for the elastic deformation of the base (120) consisted of an helical spring or a fixed beam.

10. The capsule of claim 1, comprising means for the reversible fixation of the housing to the base.

Fig. 1

Fig. 2

Fig. 3

Fig. 4

Fig. 5

Fig. 6

Fig. 7a

Fig. 7b

Fig. 8a

Fig. 8b

Fig. 9a     Fig. 9b     Fig. 9c

Fig. 10a     Fig. 10b     Fig. 10c

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070088397 A1
- WO 2007047681 A2
- US 20060136004 A1
- US 6984902 B
- US 7843090 B
- US 7729768 B
- US 3456134 A
- US 20100217364 A
- US 20070293904 A1
- US 20070293904 A
- US 20100176664 A